# EUROPÄISCHE PATENTSCHRIFT

(11) **EP 0 936 223 B1**
(45) Veröffentlichungstag und Bekanntmachung des Hinweises auf die Patenterteilung: **05.11.2003**
(21) Anmeldenummer: 99101950.6
(22) Anmeldetag: 30.01.1999
(51) Int. Cl.: C08F 8/00, C08K 3/36, A61L 15/00

(54) **Modifizierte Superabsorber auf Basis von Polyacrylnitril-Emulsionen**
Modified superabsorbant based on polyacrylonitrile emulsions
Matériau superabsorbant à base d'émulsions de polyacrylonitrile

(30) Priorität: 11.02.1998 DE 19805447
(43) Veröffentlichungstag der Anmeldung: 18.08.1999
(73) Patentinhaber: BAYER AG, 51368 Leverkusen (DE)
(72) Erfinder: Schapowalov, Sergej, Dr., 2640 Mortsel (BE); Sackmann, Günter, Dr., 51379 Leverkusen (DE); Meyer, Rolf-Volker, Dr., 53804 Much (DE); Korte, Siegfried, Dr., 51519 Odenthal (DE)

(56) Entgegenhaltungen:
- EP-A- 0 241 885
- EP-A- 0 629 411
- EP-A- 0 783 005
- DE-A- 4 040 369
- DE-C- 716 322
- FR-A- 2 345 470
- GB-A- 2 094 809
- DATABASE WPI Section Ch, Week 9649 Derwent Publications Ltd., London, GB; Class A35, AN 96-493491 XP002103734 & JP 08 253597 A (NIPPON SYNTHETIC CHEM IND CO), 1. Oktober 1996
- CHEMICAL ABSTRACTS, vol. 110, no. 8, 20. Februar 1989 Columbus, Ohio, US; abstract no. 58375g, Seite 20; XP000158099 & CN 86 104 111 A (CHINA PETROCHEMICAL CORP.)

## Beschreibung

Die vorliegende Erfindung betrifft superabsorbierende Polymerisate auf Basis von feinteiligen vernetzten oder unvernetzten Polyacrylnitril-Emulsionen und ihre verbesserten anwendungstechnischen Eigenschaften.

Superabsorbierende Polymerisate sind bekannt und werden hauptsächlich bei der Herstellung von Windeln und Inkontinenzartikeln, aber auch als wasserspeichernde Materialien in der Landwirtschaft sowie zur Ummantelung von Elektrokabeln eingesetzt. In der Regel handelt es sich bei den kommerziell verfügbaren superabsorbierenden Polymerisaten um weitmaschig vernetzte, wasserunlösliche Polymerisate auf Basis von Alkalisalzen der Polyacrylsäure oder von Copolymerisaten aus Acrylsäure und Acrylamid, die durch radikalisch initiierte Copolymerisation von Acrylsäure und polyfunktionellen Monomeren, wi z. B. Divinylbenzol, Ethylenglykoldimethacrylat, Ethylenglykoldiallylether, Butandiolacrylat, Hexandiolmethacrylat, Polyglykoldiacrylat, Trimethylolpropandiacrylat, Allylacrylat, Diallylacrylamid, Triallylamin, Diallylether, Methylenbisacrylamid und N-Methylolacrylamid, erhalten werden. Aufgrund ihrer molekularen Struktur sind solche Polymerisate in der Lage, unter Quellung und Ausbildung von Hydrogelen große Mengen an Flüssigkeiten aufzunehmen und diese auch unter Druck festzuhalten.

Aus der europäischen Patentanmeldung EP-A 0 670 335 ist bekannt, daß durch eine partielle alkalische Hydrolyse von feinteiligen wäßrigen Emulsionen von linearen Homo- und/oder Copolymerisaten des Acrylnitrils Produkte mit ausgezeichneten superabsorbierenden Eigenschaften hergestellt werden können, worin 30 bis 60 Mol-% der Nitrilgruppen in Carboxylatgruppen, 20 bis 60 Mol-% der Nitrilgruppen in Carbonamidgruppen umgewandelt wurden und 10 bis 20 Mol-% der Nitrilgruppen unverändert geblieben sind. Diese unvemetzten superabsorbierenden Polymerisate zeigen ein außerordentlich hohes Quellvermögen von bis zu 1.000 g/g in Wasser und bis zu 90 g/g in physiologischer Natriumchlorid-Lösung. Aus der EP-A 0 697 416 ist weiterhin bekannt, daß durch eine partielle alkalische Hydrolyse von feinteiligen wäßrigen Emulsionen von durch den Einbau polyfunktioneller Monomere schwach vernetzten Homo- und/oder Copolymerisaten des Acrylnitrils ebenfalls superabsorbierende Polymerisate mit ausgezeichneten anwendungstechnischen Eigenschaften erhalten werden können. Die aus vernetzten Polyacrylnitril-Emulsionen hergestellten superabsorbierenden Polymerisate, deren Nitrilgruppen zu 30 bis 80 Mol-% in Carboxylatgruppen, zu 20 bis 70 Mol-% in Carbonamidgruppen umgewandelt wurden und die noch zwischen 0 und 20 Mol-% unveränderte Nitrilgruppen enthalten, können bis zu 700 g/g an Wasser und bis zu 60 g/g an physiologischer Natriumchlorid-Lösung aufnehmen.

Die zur Herstellung der superabsorbierenden Polymerisate benötigten feinteiligen wäßrigen unvernetzten oder vernetzten Polyacrylnitril-Emulsionen werden durch die Homo- und/oder Copolymerisation von Acrylnitril in Gegenwart spezieller anionischer polymerer Emulgatoren erhalten (DE-OS 42 33 026). Die Molekulargewichte der nach diesem Verfahren dargestellten unvernetzten Polyacrylnitril-Emulsionen liegen im Bereich von 5·10⁵ bis 1·10⁷ g/Mol, bevorzugt von 2·10⁶ bis 5·10⁶ g/Mol. Die Teilchengrößen der unvernetzten oder vernetzten wäßrigen Polyacrylnitril-Emulsionen liegen im Bereich zwischen 100 und 300 nm, bevorzugt zwischen 100 und 200 nm (bestimmt mittels Laser Korrelations-Spektroskopie).

Allerdings weisen die durch die partielle alkalische Hydrolyse von unvernetzten oder vernetzten wäßrigen Polyacrylnitril-Emulsionen darstellbaren superabsorbierenden Polymerisate auch einige nachteilige Eigenschaften auf, unter denen vor allem eine gewisse Neigung zum sogenannten "Gelblocking" zu nennen ist. Das "Gelblocking" führt beim Einsatz dieser Produkte in Hygieneartikeln, wie z. B. Babywindeln, zu einer niedrigen Anfangstransportgeschwindigkeit der aufzunehmenden Flüssigkeit, was insgesamt zu einem relativ schlechten Flüssigkeitstransport führt, sowie zu einer geringen "Absorbency Under Load", d. h. unter einer hohen Druckbelastung von z.B. 0,7 psi nimmt die Absorption der superabsorbierenden Teilchen merklich ab. Ein solches Verhalten führt dann schließlich auch zu niedrigen "Rewet"-Werten der Windeln.

Dieses "Gelblocking"-Verhalten ist auch bei Superabsorbern auf Basis von Polyacrylsäure bekannt. Zur Vermeidung bzw. Behebung dieser für die Performance der Produkte negativen Eigenschaft können die Superabsorber z.B. mit Antiblockingmitteln, wie z.B. Kieselgel, Cellulose-Fluff oder anderen synthetischen oder natürlichen hydrophilen Fasern oder auch Materialien, die eine größere Oberfläche als die Superabsorber selbst aufweisen, behandelt werden (s.a. DE-OS 44 42 606). Ein weiteres Verfahren zur Vermeidung des "Gelblocking"-Verhaltens von Superabsorbern stellt eine Oberflächenvernetzung der superabsorbierenden Teilchen mit verschiedenen Vernetzungsmitteln, die mindestens zwei funktionelle Gruppen aufweisen, dar. So sind z.B. in der DE-OS 40 20 780 verschiedene Alkylencarbonate, wie 1,3-Dioxolan-2-on oder 4-Methyl-1 ,3-dioxolan-2-on, in der DE-OS 44 42 605 Aldehyde oder auch bifunktionelle Isocyanate als effektive Vernetzungsmittel genannt. Allen den dort aufgeführten Verfahren ist gemeinsam, daß nach der Oberflächenvernetzung eine Temperaturbehandlung der so modifizierten Superabsorber bei Temperaturen zwischen 150 und 300°C erfolgen muß, um eine effektive Verhinderung des "Gelblocking" zu erreichen.

Der vorliegenden Erfindung lag die Aufgabe zugrunde, das "Gelblocking" zu vermeiden und damit eine Erhöhung der Anfangstransportgeschwindigkeit, eine Verbesserung der "Absorbency Under Load" und der Transportgeschwindigkeit unter Druck zu erreichen. Dieses Ziel wurde erfindungsgemäß dadurch erreicht, daß eine Oberflächenvernetzung mit bifunktionellen Verbindungen und eine gleichzeitige Immobilisierung von Kieselsäure in der Oberflächenstruktur der superabsorbierenden Polymerisate durchgeführt wurde.

Überraschenderweise wurde gefunden, daß durch die gleichzeitig stattfindende Vernetzung und die Immobilisierung von Kieselsäure ein synergistischer Effekt eintritt. Dies zeigt sich in einem Verschwinden des "Gelblocking" bei gleichzeitiger Erhöhung der Anfangstransportgeschwindigkeit sowie einer deutlichen Verbesserung der "Absorbency Under Load"-Messwerte bei 0,3 und 0,7 psi.

Die Modifizierung der Oberfläche, welche man auch als Immobilisierung bezeichnen kann, erfolgt an den fertig ausgebildeten Superabsorber-Teilchen in einem Wasser/-Alkohol-Gemisch mit einer Wasserkonzentration von 2 bis 15 Gew.-%, bevorzugt 6 bis 12 Gew.-%, mit Formaldehyd oder anderen Aldehyden, z. B. Glutaraldehyd, als Vernetzungsmitteln in Gegenwart von Kieselsäure. Die Konzentration an Formaldehyd in dem zur Modifizierung der Superabsorber eingesetzten Reaktionsgemisch beträgt dabei 0,1 bis 2,0 Gew.-%, bevorzugt 0,3 bis 1,0 Gew.-%, und die Konzentration an Kieselsäure liegt zwischen 0,3 und 2,0 Gew.-%, bevorzugt 0,5 bis 1,5 Gew.-%. Als Alkohole können Methanol, Ethanol, n-Propanol, i-Propanol oder auch Gemische der genannten Alkohole, jedoch bevorzugt Methanol oder Ethanol, bei einem Flottenverhältnis von 0,1 bis 10, bevorzugt 0,2 bis 1, und einem Feststoffgehalt nach der Filtration vor dem Trocknen von 50 bis 90 Gew.-%, bevorzugt 70 bis 85 Gew.-%, eingesetzt werden. Die Trocknungstemperatur für die mit dem oben genannten Reaktionsgemisch behandelten superabsorbierenden Polymerisate liegt bei 60 bis 120°C, bevorzugt bei 80 bis 100°C. Die Konzentration des Wassers im Reaktionsgemisch ist abhängig vom Flottenverhältnis. Liegen die Wassergehalte oberhalb von 15 %, dann kommt es zu einer Agglomeration von Sekundärteilchen bei der Trocknung. Die gemäß der Erfindung erhaltenen superabsorbierenden Polymeren sind bevorzugt dadurch charakterisiert, dass sie als Ergebnis des Verfahrens 0,03 bis 0,6 Gew.-% Aldehyd, 0,05 bis 0,8 Gew.-% Kieselsäure und 0,5 bis 8,0 Gew.-% Wasser enthalten, wobei sich die Prozentgehalte auf das Gewicht des superabsorbierenden Polymeren beziehen. Die verwendete Kieselsäure weist bevorzugt eine Teilehengrösse von 10nm bis 50nm auf.

Die erfindungsgemäßen superabsorbierenden Polymerisate werden beispielsweise in Hygieneprodukten, wie z.B. Babywindeln und Inkontinenzartikeln, als wasserspeichemde Materialien in der Landwirtschaft oder bei der Ummantelung von Elektrokabeln eingesetzt. Gegenstand der Anmeldung ist die Verwendung der erfindungsgemäßen superabsorbierenden Polymerisate in Hygieneprodukten, als wasserspeichemde Materialien in der Landwirtschaft oder bei der Ummantelung von Elektrokabeln.

### Beispiele

### Herstellung der Hydrolyseprodukte

Als Ausgangsprodukte, an denen die Modifizierungen durchgeführt werden, wurden die nachfolgend beschriebenen Produkte A bis E eingesetzt. Deren Herstellung erfolgte durch alkalische Hydrolyse mit wässriger NaOH-Lösung.

### Produkt A:

In einem 2 1-Rührgefäß mit Rückflußkühler, Tropftrichter, Thermometer und Stickstoffeinlaß wurden bei Raumtemperatur 963,6 g einer mit 0,75 Gew.-%, bezogen auf Acrylnitril, vernetzten Polyacrylnitril-Emulsion mit einer mittleren Teilchengröße von 93 nm und einem Feststoffgehalt von 21,7 Gew.-% zusammen mit 529,6 g Ethanol vorgelegt. Danach tropft man unter Rühren mit einer Drehzahl von 250 U/m 177,7 g einer 45 gew.-%igen wäßrigen NaOH-Lösung zu. Beim Erhitzen auf 76 bis 78°C begann sich das Reaktionsgemisch unter NH₃-Abspaltung dunkelrot zu verfärben. Der abgespaltene Ammoniak wurde aus dem Reaktionskolben entfernt und in eine mit Wasser gefüllte Absorptionsflasche eingeleitet. Durch Titration des absorbierten NH₃ mit 1 N HCl kann der Verlauf der Reaktion quantitativ verfolgt werden. Bei einem Hydrolysegrad von 59,5 Mol-%, der nach ca. 3 Stunden erreicht war, wurde das hellgelb bis weiß gefärbte Reaktionsgemisch auf Raumtemperatur abgekühlt und die überschüssige NaOH mit wäßriger Ameisensäure neutralisiert. Durch Zugabe von 500 g Ethanol wurde das Reaktionsprodukt ausgefällt und die entstandene Suspension abfiltriert und auf der Filternutsche mit einem Wasser/Ethanol-Gemisch gewaschen. Nach dem Trocknen im Trockenschrank bei 65°C wurde das Produkt gemahlen und durch Siebung klassiert. Die Durchmesser der Teilchen lagen zwischen 100 und 800 µm.

### Produkt B:

Hier wurde eine unvernetzte Polyacrylnitril-Emulsion mit einem [η]-Wert von 9,02 (dl/g) - gemessen in DMF mit Elektrolytzusatz -, einer mittleren Teilchengröße von 82 nm und einem Feststoffgehalt von 20,0 Gew.-% zur Hydrolyse eingesetzt. Die

Reaktion wurde unter folgenden Bedingungen durchgführt: PAN-Konzentration im Reaktionsgemisch: 11,0 Gew.-%, NaOH-Konzentration im Reaktionsgemisch: 5,81 Gew.-%, das bedeutet ein molares Verhältnis von PAN:NaOH von 1:0,7. Die Reaktion erfolgte in Wasser ohne Zusatz von Ethanol bei 95°C. Nach einer Verweilzeit von 1,5 Stunden betrug der Hydrolysegrad 57,7 Mol-%.

### Produkt C:

Produkt C wurde aus Produkt B erhalten, nachdem dieses einer 15-minütigen thermischen Behandlung bei 180°C unterworfen worden war.

### Produkt D:

Zur Herstellung von Produkt D wurde dasselbe Verfahren wie für Produkt A eingesetzt. Die dabei zur Hydrolyse verwendete unvernetzte Polyacrylnitril-Emulsion besaß einen Feststoffgehalt von 31,5 Gew.-%, eine mittlere Teilchengröße von 115 nm sowie einen [η]-Wert von 9,32 (dl/g). Der Hydrolysegrad betrug nach einer Verweilzeit von 2,25 Stunden 54,0 Mol-%

### Produkt E:

Die Herstellung von Produkt E erfolgte nach demselben Verfahren wie die der. Produkte A und D ebenfalls in einem Wasser/Ethanol-Gemisch. Die eingesetzte unvernetzte Polyacrylnitril-Emulsion hatte einen Feststoffgehalt von 30,0 Gew.-%, einen [η]-Wert von 9,2 (dl/g) und eine mittlere Teilchengröße von 120 nm. Nach einer Verweilzeit von 3 Stunden betrug der Hydrolysegrad ca. 63 Mol-%.

### Modifizierung der Hydrolyseprodukte

Die Modifizierung der Hydrolyseprodukte A bis E, deren Teilchendurchmesser im Bereich von 100 und 800 µm lag, erfolgte nach folgender allgemeiner Vorschrift:

### Beispiel 1:

35 g des Produktes A wurden mit 200 g eines Reaktionsgemischs, das folgende Zusammensetzung aufwies, 20 Minuten bei Raumtemperatur gerührt:
178,0 g Methanol
18,0 g entionisiertes Wasser
3,0 g Kieselsäure
1,0 g Formaldehyd

Nach der Filtration über eine Nutsche wurde das Rohprodukt mit einem Feststoffgehalt von 70,1 Gew.-% 30 Minuten bei 98°C im Umluftschrank getrocknet.

### Vergleichsbeispiele I-IV:

Durch die Vergleichsbeispiele I bis IV soll der synergistische Effekt nachgewiesen werden, der durch die gleichzeitig stattfindende Vernetzung und die Immobilisierung von Kieselsäure eintritt.

### Vergleichsbeispiel I:

35 g des Produktes A, das eine definierte Teilchengrößenverteilung aufwies, wurden mit 200 g eines Reaktionsgemischs folgender Zusammensetzung 20 Minuten bei R.T. gerührt:
182,0 g Methanol
18,0 g entionisiertes Wasser

Nach der Filtration über eine Nutsche wurde das Rohprodukt mit einem Feststoffgehalt von 70,1 Gew.-% 45 Minuten bei 98°C getrocknet.

### Vergleichsbeispiel II:

Behandlung (20 Minuten bei Raumtemperatur) von 35 g des Produktes A mit 200 g eines Reaktionsgemischs folgender Zusammensetzung:
179,0 g Methanol
18,0 g entionisiertes Wasser
3,0 g Kieselsäure

Feststoffgehalt nach der Filtration: 70,1% Gew.-%; Trocknung bei 98°C (45 Minuten).

### Vergleichsbeispiel III:

35 g des Produktes A wurden mit folgendem Reaktionsgemisch 20 Minuten bei Raumtemperatur behandelt:
181,0 g Methanol
18,0 g entionisiertes Wasser
1,0 g Formaldehyd

Feststoffgehalt nach der Filtration: 70,2 Gew.-%; Trocknung bei 98°C (45 Minuten).

### Vergleichsbeispiel IV:

20 g des Produktes aus Vergleichsbeispiel III wurden mit 114,8 g eines Reaktionsgemischs mit folgender Zusammensetzung 20 Minuten bei R.T. gerührt:
182,8 g Methanol
10,4 g entionisiertes Wasser
1,8 g Kieselsäure

Nach der Filtration betrug der Feststoffgehalt 70,3%; die Trocknung erfolgte bei 98°C über 45 Minuten.

### Beispiel 2:

100 g von Produkt B wurden mit 570 g eines Reaktionsgemischs folgender Zusammensetzung 20 Minuten bei Raumtemperatur gerührt:
507,3 g Methanol
51,3 g entionisiertes Wasser
8,0 g Kieselsäure
3,4 g Formaldehyd

Nach der Filtration wurde das modifizierte Hydrolysat mit einem Feststoffgehalt von 70,3 Gew.-% 45 Minuten bei 98°C getrocknet.

### Beispiel 3:

Beispiel 3 wurde nach demselben Verfahren wie Beispiel 2 durchgeführt mit dem Unterschied, daß anstelle von Produkt B das Produkt C zur Modifizierung eingesetzt wurde.

### Beispiel 4:

100 g von Produkt D werden mit 570 g eines Reaktionsgemischs nachfolgender Zusammensetzung 5 Minuten bei Raumtemperatur zur Reaktion gebracht:
507,3 g Methanol
51,3 g entionisiertes Wasser
8,6 g Kieselsäure
2,8 g Formaldehyd

Nach der Filtration wurde das Rohprodukt mit einem Feststoffgehalt von 70,3 Gew. % 45 Minuten bei 98°C getrocknet.

### Beispiel 5:

1.025 g von Produkt E wurden mit einem Reaktionsgemisch folgender Zusammensetzung 10 Minuten bei Raumtemperatur gerührt:
1.368,8 g Ethanol
138,4 g entionisiertes Wasser
15,4 g Kieselsäure
15,4 Formaldehyd

Nach der Filtration wurde das modifizierte Produkt mit einem Feststoffgehalt von 75,9 Gew.-% 2 Stunden bei 90°C getrocknet.

In der nachfolgenden Tabelle 1 sind die anwendungstechnischen Eigenschaften der nach den Beispielen 1 bis 5 erhaltenen modifizierten Superabsorber zusammengestellt. Gemessen wurden dabei folgende Eigenschaften:
⇒ Absorption nach der Zylinder-Methode wie sie in DE-OS 40 15 085 beschrieben ist; dabei wurden die Meßwerte alle 2 Sekunden on-line genommen. In der Tabelle 1 sind jedoch nur die Werte nach 15 Sekunden, 3 Minuten, 10 Minuten und 30 Minuten aufgeführt.
⇒ Absorption nach der Teebeutel-Methode (gemäß "edana" 440.0-96)
⇒ Retention (C.C.); (gemäß "edana" 441.0-96)
⇒ AUL (Absorbency Under Load) bei 0,3 psi und 0,7 psi (gemäß "edana" 442.0-96
⇒ Anfangsabsorptionsgeschwindigkeit - berechnet aus den linearen Abschnitten der Absorptionskurven

Außerdem sind in der Tabelle die wasserlöslichen Anteile (WLA), die durch Extraktion und gravimetrische Bestimmung der Eindampfrückstände bestimmt wurden sowie die pH-Werte angegeben. Alle Untersuchungen wurden mit 0,9%-iger NaCl-Lösung durchgeführt.

Als Vergleiche zeigt die Tabelle ebenfalls die Beispiele 1A bis 5E. hierbei handelt es sich jeweils um die nicht modifizierten Produkte A bis E, aus denen die Beispiele 1 bis 5 hergestellt wurden.

In den vier letzten Zeilen der **Tabelle 1** sind zusätzlich noch die Ergebnisse eingetragen, die mit den Vergleichsbeispiele I bis IV erhalten wurden.

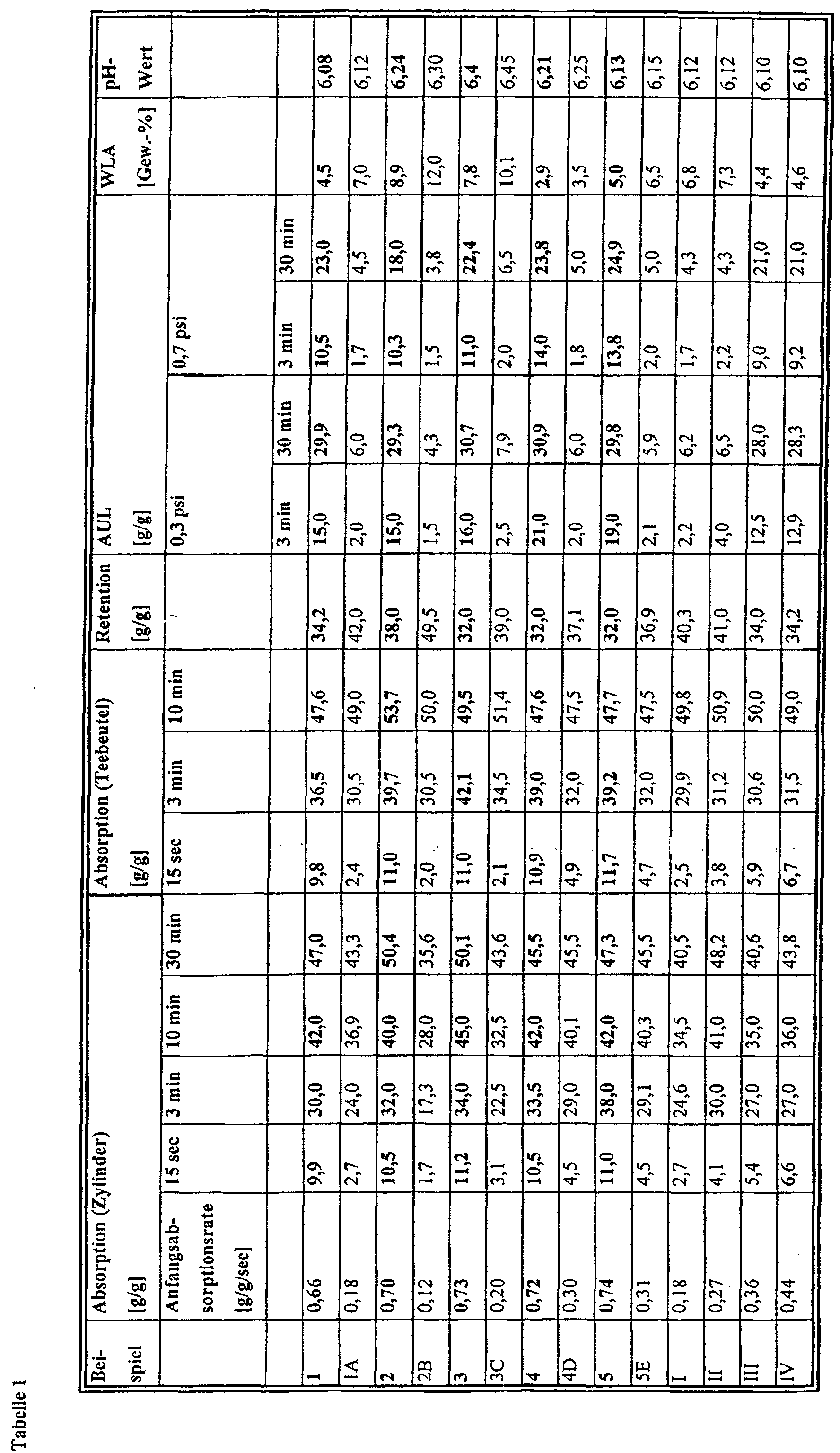

Aus den in Tabelle 1 zusammengefassten Ergebnissen geht ganz klar hervor, daß durch die erfindungsgemäße Modifizierung sowohl die anfänglichen Absorptionsgeschwindigkeiten (s. Spalte 2 der Tabelle) - gemessen sowohl nach der Zylinder- als auch nach der Teebeutel-Methode - als auch die AUL-Werte im Vergleich zu den nicht modifizierten Superabsorbern stark erhöht werden. Außerdem liegen die Werte für die wasserlöslichen Anteile in den modifizierten Produkten wesentlich niedriger als in den unmodifizierten.

Die Ergebnisse der mit den nach den Vergleichsbeispielen I bis IV erhaltenen Produkten lassen sehr deutlich erkennen, daß die gleichzeitige Modifizierung mit Kieselsäure und Formaldehyd zu einer gleichzeitigen Erhöhung der Anfangstransportgeschwindigkeit unter Druck und der AUL-Werte führt (Vergleich zwischen I-IV und Beispiel 1). Wie weiterhin der Vergleich zwischen III und IV sowie Beispiel 1 zeigt, führt die gleichzeitige Vernetzung und Immobilisierung von Kieselsäure nicht nur zur Erhöhung der AUL-Werte sondern auch zu einer Erhöhung der Anfangstransportgeschwindigkeit mit und ohne Druck und zu einer Verminderung des Gelblocking-Effektes.

### Ergebnisse von Rewet-Messungen

An den nach den Beispielen 2, 3 und 5 hergestellten superabsorbierenden Polymerisaten wurden Rewet-Messungen im Vergleich zu den nicht-modifizierten Beispielen 2B, 3C und 5E durchgeführt.

Rewet-Messungen dienen zum Nachweis, daß die superabsorbierenden Polymerisate unter praxisnahen Bedingungen, wie z. B. beim Einsatz in Babywindeln, Körperflüssigkeiten auch unter Druck festzuhalten vermögen. Dabei werden in einem Zellstoff-Fluff mit der Fläche 13×21 cm 5 g Superabsorber gleichmäßig verteilt und dann mit 70 ml einer 0,9%igen NaCl-Lösung begossen. Danach deckt man die Fläche mit einem Stapel Filterpapier ab und belastet 15 sec mit einem Gewicht von 4 kg. Nach Messung der Gewichtszunahme der Filterpapiere wird die Prozedur - Aufgabe von 70 ml Flüssigkeit und erneute Belastung mit 4 kg - noch zweimal wiederholt. Die nach jedem Prüfvorgang gemessene Gewichtszunahme der Filterpapiere stellt ein Maß für die Fähigkeit der Superabsorber dar, Flüssigkeit auch unter großer Belastung festzuhalten.

In der nachfolgenden Tabelle 2 sind die erhaltenen Messergebnisse zusammengefasst.

**Tabelle 2**

| **Beispiel** | **Rewet** **[g]** | | |
|---|---|---|---|
| | **1. Messung** | **2. Messung** | **3. Messung** |
| 2B | 0,05 | 2,04 | 4,19 |
| **2** | **0,04** | **0,38** | **1,21** |
| 3C | 0,05 | 1,94 | 3,67 |
| **3** | **0,04** | **0,18** | **0,75** |
| 5E | 0,04 | 1,50 | 3,40 |
| **5** | **0,02** | **0,09** | **0,39** |

Man erkennt deutlich, daß durch die Modifizierung vor allem nach der 2. und 3. Messung signifikant reduzierte Werte für die Flüssigkeitsabgabe unter Druck gefunden werden. Dies weist auf eine starke Verminderung bzw. Abwesenheit des "Gel-blocking" der modifizierten superabsorbierenden Polymerisate hin.

## Patentansprüche

1. Superabsorbierende Polymerisate auf Basis von vernetzten oder unvernetzten feinteiligen Polyacrylnitril-Emulsionen, **dadurch gekennzeichnet, dass** die Oberfläche der Polymerisatteilchen durch Vernetzung und gleichzeitige Immobilisierung von Kieselsäure in der vernetzten Oberflächenstruktur modifiziert ist und zur Oberflächenmodifizierung als Aldehyd Formaldehyd eingesetzt wird.

2. Superabsorbierende Polymerisate gemäß Anspruch 1, **dadurch gekennzeichnet, dass** die Oberflächenbehandlung mit Aldehyden in einer Konzentration von 0,03 bis 0,6 Gew-%, Kieselsäure in einer Konzentration von 0,05 bis 0,8 Gew.-% und Wasser in einer Konzentration von 0,5 bis 8,0 Gew.-%, bezogen auf das superabsorbierende Polymerisat, durchgeführt wird.

3. Superabsorbierende Polymerisate gemäß den Ansprüchen 1 bis 2, **dadurch gekennzeichnet, dass** die verwendete Kieselsäure eine Teilchengröße von 10 nm bis 50 nm aufweist.

4. Verwendung der gemäß den Ansprüchen 1 bis 3 erhältlichen superabsorbierenden Polymerisate in Hygieneprodukten, als wasserspeichernde Materialien in der Landwirtschaft oder bei der Ummantelung von Elektrokabeln.

## Claims

1. Superabsorbing polymers based on crosslinked or uncrosslinked, finely divided polyacrylonitrile emulsions, **characterised in that** the surface of the polymer particles is modified by crosslinking and simultaneous immobilisation of silica in the crosslinked surface structure and the aldehyde used for surface modification is formaldehyde.

2. Superabsorbing polymers according to claim 1, **characterised in that** the surface treatment is performed with aldehydes in a concentration of 0.03 to 0.6 wt.%, silica in a concentration of 0.05 to 0.8 wt.% and water in a concentration of 0.5 to 8.0 wt.%, relative to the superabsorbing polymer.

3. Superabsorbing polymers according to claims 1 to 2, **characterised in that** the silica used has a particle size of 10 nm to 50 nm.

4. Use of the superabsorbing polymers obtainable according to claims 1 to 3 in personal hygiene products, as water storage materials in agriculture or for sheathing electrical cables.

## Revendications

1. Polymères superabsorbants à base d'émulsions fines de polyacrylonitrile réticulé ou non, **caractérisés en ce que** la surface des particules de polymères est modifiée par réticulation et immobilisation simultanée de silice dans la structure superficielle réticulée et **en ce que** l'aldéhyde utilisé pour la modification superficielle est le formaldéhyde.

2. Polymères superabsorbants selon la revendication 1, **caractérisés en ce que** le traitement de surface a été réalisé à l'aide d'aldéhydes à une concentration de 0,03 à 0,6 % en poids, de silice à une concentration de 0,05 à 0,8 % en poids et d'eau à une concentration de 0,5 à 8,0 % en poids par rapport au polymère superabsorbant.

3. Polymères superabsorbants selon les revendications 1 à 2, **caractérisés en ce que** la silice utilisée est à une dimension de particule de 10 nm à 50 nm.

4. Utilisation des polymères superabsorbants obtenus selon les revendications 1 à 3 dans des articles d'hygiène, dans des matériaux réserves d'eau pour l'agriculture ou pour des gaines de câbles électriques.
